(19) Europäisches Patentamt
European Patent Office
Office européen des brevets

(11) **EP 4 450 053 A1**

(12) **DEMANDE DE BREVET EUROPEEN**

(43) Date de publication:
**23.10.2024 Bulletin 2024/43**

(21) Numéro de dépôt: **23305595.3**

(22) Date de dépôt: **18.04.2023**

(51) Classification Internationale des Brevets (IPC):
***A61K 8/365** (2006.01)* ***A61K 8/49** (2006.01)*
***A61K 8/73** (2006.01)* ***A61K 8/9728** (2017.01)*
***A61Q 1/02** (2006.01)* ***A61Q 19/00** (2006.01)*

(52) Classification Coopérative des Brevets (CPC):
**A61Q 1/02; A61K 8/365; A61K 8/4973; A61K 8/736; A61K 8/9728; A61Q 19/00;**
A61K 2800/43

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**KH MA MD TN**

(71) Demandeur: **L'OREAL**
**75008 Paris (FR)**

(72) Inventeurs:
- **BOILEAU, Nathalie**
**94550 Chevilly Larue (FR)**
- **KUSINA, Christophe**
**94550 Chevilly Larue (FR)**

(74) Mandataire: **Lavoix**
**2, place d'Estienne d'Orves**
**75441 Paris Cedex 09 (FR)**

(54) **COMPOSITION À BASE DE CHITOSAN ET DE L'ACIDE SPICULISPORIQUE OU SES SELS**

(57) La présente invention concerne une composition cosmétique, notamment de maquillage et/ou de soin de la peau et/ou des lèvres, en particulier des lèvres, comprenant, dans un milieu aqueux physiologiquement acceptable :

a) au moins 0,01% en poids par rapport au poids total de composition de chitosan natif ayant un poids moléculaire strictement supérieur à 3000 Daltons, cette quantité étant strictement inférieure à 15% en poids, et
b) de l'acide spiculisporique et/ou ses sels.

EP 4 450 053 A1

**Description**

La présente invention concerne une composition cosmétique comprenant, dans un milieu aqueux physiologiquement acceptable :

a) au moins 0,01% en poids par rapport au poids total de composition de chitosan natif ayant un poids moléculaire strictement supérieur à 3000 Daltons, cette quantité étant strictement inférieure à 15% en poids, et
b) de l'acide spiculisporique et/ou ses sels.

Elle se rapporte également à un procédé de maquillage et/ou de soin de la peau et/ou des phanères, dans lequel on applique la composition selon l'invention sur la peau et/ou les phanères.

Il existe de nombreuses compositions cosmétiques pour lesquelles des propriétés de tenue du film déposé, après application sur les matières kératiniques, sont souhaitées. On peut citer par exemple les rouges à lèvres ou les vernis à ongles. Afin d'obtenir un tel résultat, il est possible d'associer des matières premières particulières, notamment des agents filmogènes. Cependant, la présence de tels agents pour la tenue du film sur les matières kératiniques peut conduire à des compositions desséchantes.

Par ailleurs, on cherche souvent à obtenir des compositions couvrantes.

Les systèmes à base de chitosan montrent que l'association de chitosan et d'un pigment permet de générer des films résistants à la friction à sec et à l'huile. Néanmoins, la sensibilité à l'eau de ces dépôts est très forte et reste un challenge majeur des technologies à base de chitosan.

Le formulateur est donc à la recherche de matières premières et/ou de systèmes permettant d'obtenir des compositions fluides à base de chitosan (i.e. qui s'écoulent) dont le dépôt est couvrant, homogène, présentant une bonne tenue et dont la résistance à l'eau est améliorée.

Par ailleurs, la formulation de produits cosmétiques respectueux de l'environnement, c'est-à-dire dont la conception et le développement tiennent compte des enjeux environnementaux, devient une préoccupation majeure pour contribuer à relever les défis planétaires.

Il se révèle donc essentiel de proposer des compositions et/ou des procédés de préparation et/ou des ingrédients plus durables permettant ainsi de répondre à ces enjeux environnementaux.

Dans ce contexte, il est important de développer de nouvelles compositions cosmétiques avec une meilleure empreinte carbone notamment en favorisant l'emploi de matières premières renouvelables et/ou avec un bon index de naturalité et/ou d'origine naturelle et plus particulièrement d'origine végétale tout en réduisant l'utilisation de composés d'origine pétrochimique.

La présente invention a pour but de proposer des compositions cosmétiques aqueuses colorées ou non présentant de bonnes propriétés, notamment en termes d'homogénéité et de bonne tenue, en particulier ayant une bonne tenue/résistance à l'eau.

Après application, ces compositions laissent un dépôt filmogène couvrant et homogène, qui a une bonne tenue à l'usure. Les films colorés formés sont adhésifs et cohésifs, et présentent une résistance améliorée à l'eau.

Ces compositions comprennent également des ingrédients durables, permettant ainsi de répondre aux enjeux environnementaux.

La présente invention a donc pour objet une composition cosmétique, notamment de maquillage et/ou de soin de la peau et/ou des lèvres, en particulier des lèvres, comprenant, dans un milieu aqueux physiologiquement acceptable :

a) au moins 0,01% en poids par rapport au poids total de composition de chitosan natif ayant un poids moléculaire strictement supérieur à 3000 Daltons, cette quantité étant strictement inférieure à 15% en poids, et
b) de l'acide spiculisporique et/ou ses sels.

Par « physiologiquement acceptable », on entend un milieu compatible avec les matières kératiniques.

Elle a également pour objet un procédé de maquillage et/ou de soin de la peau et/ou des phanères, dans lequel on applique la composition selon l'invention sur la peau et/ou les phanères.

## Chitosan

La composition selon l'invention comprend au moins 0,01% en poids par rapport au poids total de la composition d'au moins un chitosan natif ayant un poids moléculaire strictement supérieur à 3000 Daltons (3 kDa).

La quantité de chitosan natif est également strictement inférieure à 15% en poids par rapport au poids total de la composition.

De préférence, le chitosan natif a un poids moléculaire supérieur ou égal à 10 kDa, de préférence supérieur ou égal à 15 kDa, de préférence supérieur ou égal à 20 kDa. De préférence, le chitosan natif a un poids moléculaire

compris entre 10 kDa et 2 MDa, de préférence compris entre 15 kDa et 1,5 MDa, de préférence compris entre 20 kDa et 300 kDa, de préférence compris entre 20 kDa et 200 kDa.

Le chitosan (ou chitosane) est très peu répandu dans la nature. Il n'est signalé que dans les exosquelettes de certains insectes comme les reines des termites et dans les parois cellulaires d'une classe particulière de champignons, les zygomycètes.

Le chitosan est obtenu par désacétylation de la chitine. La chitine est un polysaccharide composé de plusieurs unités N-acétyl-D-glucosamine reliées entre elles par une liaison de type β (1,4).

La structure chimique idéale du chitosane est un enchaînement de monomères β-D-glucosamine reliés par une liaison glycosidique (1→4).

Par « chitosan » selon l'invention, on entend tout copolymère formé d'unités constitutives N-acétyl-D-glucosamine et D-glucosamine, dont le degré d'acétylation est inférieur à 90%. Le chitosan est constitué des unités sucres glucosamine (unités désacétylées) et d'unités N-acétyl-D-glucosamine (unités acétylées) reliées entre elles par des liaisons de type β (1,4) et constitue un polymère du type Poly(N-acetyl-D-glucosamine) -poly(D-glucosamine).

De préférence, le degré d'acétylation du chitosan est inférieur ou égal à 80%, de préférence inférieur ou égal à 70%, de préférence inférieur ou égal à 60%, de préférence, inférieur ou égal à 50%, de préférence inférieur ou égal à 35%, de préférence inférieur ou égal à 25%, de préférence inférieur ou égal à 15%.

Le degré d'acétylation est le pourcentage d'unités acétylées par rapport au nombre d'unités totales, il peut être déterminé par spectroscopie infrarouge à transformée de Fourier (IR-TF) ou par un titrage par une base forte.

Le chitosan de l'invention est de préférence un polysaccharide préparé à partir d'une origine fongique. Il est notamment extrait et purifié à partir de sources fongiques alimentaires ou biotechnologique sûres et abondantes tels que *Agaricus bisporus* ou *Aspergillus niger.* Le chitosan de l'invention est de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus.* De préférence le champignon est *Aspergillus niger.*

Le chitosan peut être d'origine OGM, mais de préférence est d'origine non OGM.

Le chitosan selon l'invention est natif, c'est-à-dire qu'il n'est pas modifié. Il ne contient notamment pas de modification chimique.

Une méthode de préparation du chitosan est celle décrite dans la demande WO03068824.

De préférence, le chitosan utilisé dans l'invention est sous forme de poudre. Il est notamment commercialisé par Kitozyme sous le nom Kiosmetine ou Kionutrime.

Le chitosan est de préférence présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

## Acide spiculisporique

La composition selon l'invention comprend de l'acide spiculisporique également connu sous le nom de 4,5-dicarboxy-4-pentadécanolide, de formule (I) :

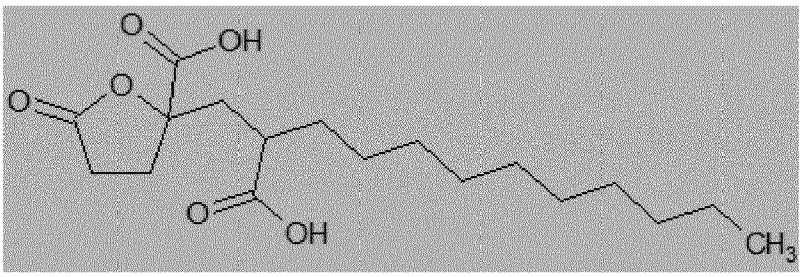
(I)

L'acide spiculisporique (encore appelé « acide-S ») susceptible d'être employé dans le cadre de l'invention peut être sous forme salifiée ou non. Lorsqu'il se présente sous forme d'un sel, il peut s'agir d'un sel organique ou minéral. Dans ce cas, l'acide spiculisporique peut être employé en mélange avec une base organique ou minérale. Plus exactement, l'acide-S peut former un mono-sel, un di-sel ou un tri-sel.

Le mono-sel correspond au produit de neutralisation du groupement carboxylique lié à l'atome de carbone en position C4 de l'acide-S.

Le di-sel correspond au produit de neutralisation des groupements carboxyliques liés aux atomes de carbone en position C4 et C5 de l'acide-S.

Le tri-sel correspond au produit de neutralisation des groupements carboxyliques liés aux atomes de carbone en position C4 et C5 de l'acide S et de salification de la fonction lactone sous sa forme ouverte.

Comme exemples de bases minérales peuvent être cités les hydroxydes ou les carbonates de métal alcalin, alcalino-terreux, tels que sodium, potassium, calcium, ammonium, magnésium, lithium ou sodium. Comme exemples de base organiques peuvent être cités les amines, les alcanolamines ou les acides aminés.
La base peut être notamment choisie parmi les acides aminés et les alcanolamines. Plus particulièrement la base peut être choisie parmi les acides aminés basiques suivants l'arginine, la lysine, l'ornithine, la citruline et l'histidine, en particulier l'acide aminé est l'arginine.
La base peut aussi être choisie parmi les alcanolamines suivantes : les mono-, di- et triéthanolamines, l'isopropanolamine, le 2-amino-2-méthyl-1-propanol, ainsi que leurs mélanges. De manière préférée, l'alcanolamine est la triéthanolamine.
Dans les compositions selon l'invention, l'acide spiculisporique peut être sous forme d'un mono-sel, d'un di-sel, d'un tri-sel ou d'un mélange de plusieurs sels de degré de salification différent.
A titre d'exemple, l'acide spiculisporique est disponible sous la dénomination commerciale SPICULISPORIC ACID® de la société IWATA CHEMICAL.
L'acide-S et/ou ses sels peuvent être présents dans la composition dans une teneur allant de 0,1% à 25%, de préférence de 0,1 à 15%, de préférence de 0,1% à 10%, de préférence de 0,1% à 5%, de préférence 0,1% à 3%, de préférence 0,1% à 2%, préférentiellement de 0,25% à 1,5% et encore plus préférentiellement de 0,5% à 1,5% en poids par rapport au poids total de la composition.
Selon l'invention, le ratio pondéral entre le chitosan et l'acide spiculisporique (i.e. ratio pondéral chitosan : acide-S) est compris entre 0,1 et 10. De préférence, il est compris entre 0,2 et 10, préférentiellement entre 0,3 et 5, et encore plus préférentiellement entre 0,5 et 3.

## Matières colorantes pigmentaires

La composition selon l'invention peut comprendre en outre au moins une matière colorante pigmentaire. Cette matière colorante est choisie parmi les matières colorantes pulvérulentes comme les pigments minéraux, les nacres, les pigments organiques.
On entend par « pigments » des particules blanches ou colorées, minérales ou organiques, insolubles dans un milieu aqueux, destinées à colorer la composition et/ou le dépôt résultant.
Les matières colorantes peuvent être présentes, dans la composition, en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids.

### Pigments minéraux

Selon un mode de réalisation particulier, les pigments utilisés selon l'invention sont choisis parmi les pigments minéraux.
Par « pigment minéral », on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, le dioxyde de titane, les poudres métalliques comme la poudre d'aluminium et la poudre de cuivre. Les pigments minéraux suivants peuvent aussi être utilisés : Ta2O5, Ti3O5, Ti2O3, TiO, ZrO2 en mélange avec TiO2, ZrO2, Nb2O5, CeO2, ZnS.
La taille du pigment utile dans le cadre de la présente invention est en général supérieure à 100 nm et peut aller jusqu'à 10 μm, de préférence de 200 nm à 5 μm, et plus préférentiellement de 300 nm à 1 μm. Selon une forme particulière de l'invention, les pigments présentent une taille caractérisée par un D[50] supérieur à 100 nm et pouvant aller jusqu'à 10 μm, de préférence de 200 nm à 5μm, et plus préférentiellement de 300 nm à 1 μm. Les tailles sont mesurées par diffusion statique de la lumière au moyen d'un granulomètre commercial de type MasterSizer 3000® de chez Malvern, permettant d'appréhender la répartition granulométrique de l'ensemble des particules sur une large gamme pouvant aller de 0,01 μm à 1000 μm. Les données sont traitées sur la base de la théorie classique de diffusion de Mie. Cette théorie est la plus adaptée pour des distributions de taille allant du submicronique au multi-micronique, elle permet de déterminer un diamètre « effectif » de particules. Cette théorie est notamment décrite dans l'ouvrage de Van de Hulst, H.C., « Light Scattering by Small Particles », Chapitres 9 et 10, Wiley, New York, 1957. D[50] représente la taille maximale que présente 50 % en volume les particules.
Dans le cadre de la présente invention, les pigments minéraux sont plus particulièrement l'oxyde de fer et/ou le dioxyde de titane.
Comme pigments minéraux utilisables dans l'invention, on peut également citer les nacres. Par « nacres », il faut comprendre des particules colorées de toute forme, irisées ou non, notamment, produites par certains mollusques

dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques. On peut également citer, à titre d'exemple de nacres, le mica naturel recouvert d'oxyde de titane, d'oxyde de fer, de pigment naturel ou d'oxychlorure de bismuth. Les nacres peuvent plus particulièrement posséder une couleur ou un reflet jaune, rose, rouge, bronze, orangé, brun, or et/ou cuivré.

Parmi les pigments utilisables selon l'invention, on peut également citer ceux à effet optique différent d'un simple effet de teinte conventionnel, c'est-à-dire unifié et stabilisé tel que produit par les matières colorantes classiques, comme par exemple, les pigments monochromatiques.

Au sens de l'invention, « stabilisé » signifie dénué d'effet de variabilité de la couleur avec l'angle d'observation ou encore en réponse à un changement de température. Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Selon un mode particulier, la composition selon l'invention comprend au moins un pigment non enrobé.

## Pigments organiques

Selon un autre mode de réalisation de l'invention, la matière colorante pigmentaire est un pigment organique, synthétique, naturel ou d'origine naturelle.

Par « pigment organique », on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, et les pigments obtenus par polymérisation oxydante de dérivés indoliques, phénoliques tels qu'ils sont décrits dans le brevet FR 2 679 771.

Les pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque.

Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090). A titre d'exemples de laques, on peut citer le produit connu sous la dénomination D&C Red 7 (CI 15 850 :1).

## Charges

La composition selon l'invention peut comprendre également au moins une charge.

Par « charges », il faut comprendre des particules de toute forme, incolores ou blanches, minérales ou de synthèses, insolubles et dispersées dans le milieu de la composition quelle que soit la température à laquelle la composition est fabriquée. De manière générale, les charges comprises dans les compositions selon l'invention ne sont pas des matières colorantes pigmentaires.

De préférence, la charge est choisie parmi les particules de cellulose, les silices, l'amidon, le kaolin, les argiles, les particules de nylon ou de polyméthacrylate de méthyle (PMMA) et leurs mélanges.

Les particules de cellulose utilisables selon l'invention sont de préférence sphériques (billes de cellulose).

Par particules sphériques au sens de la présente invention, on entend des particules pleines ou poreuses ayant un paramètre de circularité d'au moins 0,95. Le paramètre de circularité est défini comme le rapport de la circonférence d'un disque ayant la même aire que la particule au périmètre de la particule. Une valeur de 1 caractérise des particules parfaitement sphériques.

Elles présentent de préférence une taille moyenne inférieure à 40 μm, de préférence allant de 1 à 20 μm, encore préférentiellement de 2 à 10 μm.

Parmi les particules de cellulose utilisables selon l'invention, on peut citer en particulier celles vendues par la société Daito sous la marque CELLULOBEADS® telles que CELLULOBEADS USE® (D[50] = 4 μm), CELLULOBEADS D-5® (D[50] < 10 μm), CELLULOBEADS D-10® (D[50] < 15 μm), CELLULOBEADS D-30® (D[50] < 30 μm).

De préférence, les charges sont présentes dans une teneur allant de 0,1% à 20% en poids par rapport au poids total de la composition, de préférence de 0,2% à 15% en poids, par rapport au poids de la composition, de préférence de 0,3% à 10% en poids, de préférence de 0,4% à 5% en poids, de préférence de 0,5% à 4% en poids.

**Alpha hydroxy acide (AHA)**

La composition selon l'invention peut comprendre au moins un AHA.

Par alpha hydroxy acide, on entend selon la présente invention un acide carboxylique ayant au moins une fonction hydroxy occupant une position alpha sur ledit acide (carbone adjacent à une fonction acide carboxylique). Cet acide peut se présenter dans la composition finale sous forme d'acide libre et/ou sous la forme de l'un de ses sels associés (sels avec une base organique ou un alcalin notamment), en particulier selon le pH final imposé à la composition.

Les α-hydroxyacides (alpha hydroxyacides ou AHA) sont par exemple choisis parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

Selon un mode préféré, l'alpha hydroxyacide est choisi parmi l'acide lactique, l'acide citrique, l'acide malique, l'acide tartrique et leurs sels. Plus particulièrement, l'alpha hydroxyacide est choisi parmi l'acide lactique, l'acide citrique, leurs sels et leurs mélanges.

Le ou les alpha hydroxyacides peuvent être présents en une quantité allant de 0,001 à 10% en poids, de 0,005 à 5% en poids, de préférence de 0,01 à 3% en poids par rapport au poids total de la composition.

**Milieu aqueux physiologiquement acceptable**

La composition selon l'invention comprend un milieu aqueux physiologiquement acceptable. Ledit milieu comprend de l'eau.

L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle.

La composition comprend de préférence au moins 5% en poids d'eau par rapport au poids total de la composition, de préférence au moins 10% en poids, de préférence au moins 20% en poids, de préférence au moins 30% en poids, de préférence au moins 40% en poids. La composition comprend de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50%.

La phase aqueuse peut également comprendre au moins un solvant organique miscible dans l'eau à 25°C.

De préférence, le solvant organique miscible dans l'eau est choisi parmi les alcools, les polyols et leurs mélanges.

Parmi les alcools, on peut citer les alcools en $C_1$-$C_{10}$, plus préférentiellement en $C_1$-$C_5$, tels que l'éthanol, l'isopropanol, le propanol et le butanol.

Le polyol est, de préférence, choisi parmi les polyols ayant de 2 à 20 atomes de carbone, plus préférentiellement de 2 à 6 atomes de carbone, comme le glycérol, le diglycérol, le propylèneglycol, l'isoprène glycol, le dipropylèneglycol,

le butylène glycol, l'hexylène glycol, le 1,2-propanediol, le 1,3-propanediol, le pentylène glycol, les polyéthylèneglycols ayant de 2 à 200 motifs d'oxyde d'éthylène et leurs mélanges.

La composition peut comprendre de 1% à 25% en poids de solvant organique miscible dans l'eau, par rapport au poids total de la composition, plus préférentiellement de 2% à 20% en poids, encore plus préférentiellement de 3% à 15% en poids.

**pH de la composition**

La composition selon l'invention présente un pH inférieur ou égal à 7, de préférence inférieur ou égal à 6,5, de préférence inférieur ou égal à 6,3. Avantageusement, le pH de la composition est compris entre 3 et 6,3, de préférence compris entre 4 et 6,3.

De préférence, la composition cosmétique selon l'invention comprend au moins une base et/ou au moins un acide. La base et l'acide selon l'invention sont connus et classiquement utilisés dans le domaine cosmétique.

La base et/ou l'acide sont notamment utilisés pour ajuster le pH final de la composition entre 3 et 6,3.

L'acide peut par exemple être l'acide citrique.

La base peut être choisie parmi les bases minérales comme par exemple les hydroxydes de métaux alcalins, l'hydroxyde de sodium, l'hydroxyde de potassium.

De préférence, la base de la composition est un hydroxyde de métal alcalin, de préférence l'hydroxyde de sodium ou l'hydroxyde de potassium.

La composition selon l'invention peut comprendre au moins une base en une teneur en matière active allant de 0,5 % à 10 % en poids, par rapport au poids total de la composition, notamment de 1 % à 5 % en poids, de préférence allant de 1 % à 4 % en poids.

La composition conforme à l'invention peut être obtenue de manière classique par l'homme du métier.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières par rapport au poids total de composition (% p/p), sauf mention contraire.

**Exemples:**

**I. Préparation d'une composition selon l'invention comprenant et comparaisons de sa résistance à l'eau avec une composition comparative**

Les compositions E, F, G et A selon l'invention, et C comparative, ont été préparées par mélange des ingrédients du tableau 1.

Les compositions A, E, F et G selon l'invention contiennent du chitosan, des pigments, et de l'acide spiculisporique dans une teneur allant de 0,25% à 1% en poids par rapport au poids total de la composition.

La composition C comparative contient du chitosan ainsi que des pigments, mais pas d'acide spiculisporique.

Puis la tenue à l'eau est évaluée. Le protocole d'évaluation est le suivant :

- Appliquer chaque composition sur une zone de chaque avant-bras (avec une zone d'avant-bras supplémentaire = témoin) : pour cela, on dépose 10µl de chaque composition sur une zone d'avant-bras de surface 2,5 x 2,5 cm pendant 15 s,
- Laisser sécher 20 min,
- Déposer 100 µL d'eau sur un tissu de frottement en coton,
- Appliquer le coton sur la zone traitée à l'aide d'un poids de 900 g pendant 5 s,
- Retirer le coton avec une force comprise entre 100 et 300 g, et
- Evaluer visuellement le dépôt témoin versus le dépôt frotté à l'eau. Cette évaluation comprend un scorage de tenue à l'eau, qui va de (-) = aucune tenue à l'eau, à (+++++) = excellente tenue à l'eau.

Les résultats sont dans le tableau 1.

[Table 1]

| Ingrédients (% p/p) | C comparative | A selon l'invention | E selon l'invention | F selon l'invention | G selon l'invention |
|---|---|---|---|---|---|
| Chitosan (Kiosmetine-CSH de Kitozyme) | 2 | 2 | 2 | 2 | 2 |

(suite)

| Ingrédients (% p/p) | C comparative | A selon l'invention | E selon l'invention | F selon l'invention | G selon l'invention |
|---|---|---|---|---|---|
| Acide lactique | 1 | 1 | 1 | 1 | 1 |
| Pigments* | 15 | 15 | 15 | 15 | 15 |
| Acide spiculisporique (IWATA CHEMICAL) | | 1 | 0,8 | 0,5 | 0,25 |
| Eau | Qsp 100 | Qsp 100 | Qsp 100 | Qsp 100 | Qsp 100 |
| **Note tenue à l'eau (de 1 (-) à 5 (+))** | 1 | 4 | 4 | 3 | 2 |
| **Formulabilité** | ok | ok | ok | ok | ok |

* Les pigments utilisés dans les compositions sont un mélange de dioxyde de titane commercialisé sous la dénomination HOMBITAN FF PHARMA-VENATOR, d'oxyde de fer rouge commercialisé sous la dénomination TAROX IRON OXIDE R-800HP - TITAN KOGYO, d'oxyde de fer jaune commercialisé sous la dénomination TAROX IRON OXIDE BL-100HPL- TITAN KOGYO et d'oxyde de fer noir commercialisé sous la dénomination TAROX IRON OXIDE LL-100HP- TITAN KOGYO.

Les résultats montrent une meilleure tenue à l'eau pour les compositions A, E, F et G selon l'invention, par rapport à la composition comparative C. L'utilisation d'acide spiculisporique, utilisé dans l'invention, apporte ainsi de la résistance à l'eau.

**II. Evaluation de la tenue à l'eau d'une composition selon l'invention comprenant une charge**

La composition J selon l'invention, et K comparative, ont été préparées par mélange des ingrédients du tableau 2.

La composition J selon l'invention contient du chitosan, de l'acide spiculisporique et des particules de silice.

La composition K comparative contient du chitosan et des particules de silice mais pas d'acide spiculisporique.

Puis la tenue à l'eau est évaluée par des mesures de Haze sur un film sec avant et après passage sous l'eau du robinet pendant 5 secondes (débit 2L/min). La mesure de Haze avant et après abrasion est faite avec la même méthode. Les performances de Haze sont comparées pour juger de l'efficacité de la composition.

***- Protocole d'étalement des compositions en un film***

L'étalement des produits se fait sur un banc d'étalement (Elcometer 4340 Applicator) permettant de régler sa vitesse ainsi que la distance sur lequel il se fait. Le banc est équipé d'un système d'aspiration relié à une pompe pour que le support où l'on fait l'étalement, ne bouge pas. Des films transparents sont utilisées (byko-chart, code 2871). L'épaisseur d'étalement est quant à elle réglable grâce à l'étaleur carré déposé sur le support de manière à étaler par arasement lorsque la plateforme est mise en marche. Chaque tranche de l'étaleur permet d'étaler avec une épaisseur différente allant de 25$\mu$m à 200$\mu$m. L'épaisseur choisie est de 25 $\mu$m afin de se rapprocher d'une épaisseur du film *in vivo.* Un poids de 960 g est ajouté par-dessus l'étaleur pendant l'étalement. La vitesse de l'étalement est réglée à 1in/sec, soit 2,54cm/s. Les films sont séchés pendant 24h à température et humidité (HR) ambiante (50% HR).

***- Protocole de mesure du Haze***

Le Haze-gard® (BYK Gardner) quantifie la perception d'effets visuels avec des mesures objectives. Il mesure l'intensité de la lumière passant au travers d'un échantillon, ce qui correspond à la transmittance hémisphérique (ou totale). Plus un matériau a une forte transmittance, et plus celui-ci possède un caractère transparent.

Le Haze-Gard® mesure de manière distincte la Transmittance Directe (i.e. dans même direction que le rayon incident) ou Hémisphérique (dans toutes les directions de l'espace, ce qui équivaut à la transmittance totale). Plus la transmittance d'un matériau est de type hémisphérique par rapport à la transmittance directe, et plus celui-ci possède un effet « flouteur ».

On définit la valeur de Haze (%) permettant de mettre en lumière ce rapport entre la transmittance hémisphérique et la transmittance directe. Ainsi plus un matériau possède une valeur de Haze élevée et plus possède un effet « flou »

(ou soft focus) élevé.

[Math 1]

$$Haze\ (\%) = 100 \times (TH - TD)/TH$$

Le dépôt de produit en une épaisseur contrôlée de 25 μm est réalisé au moyen d'un étaleur automatique (Byko drive®) sur un film plastique transparent. Après un temps de séchage de 1 heure à température ambiante et à l'air libre, les mesures des paramètres optiques (T, H, C) sont effectuées via le Haze-Gard, et ce, en trois points distincts du dépôt afin d'obtenir une moyenne. L'état du film est également caractérisé. Pour que les mesures soient pertinentes, le dépôt doit être uniforme, sans strie ou hétérogénéité.

Les résultats sont dans le tableau 2.

[Table 2]

| **Ingrédients (% p/p)** | **J selon l'invention** | **K comparative** |
|---|---|---|
| Chitosan (Kiosmetine-CSH de Kitozyme) | 2 | 2 |
| Acide lactique | 1 | 1 |
| Particules de silice (SOLESPHERE H 51) | 5 | 5 |
| Acide spiculisporique (IWATA CHEMICAL) | 1 | - |
| Eau | Qsp 100 | Qsp 100 |
| **Performance Haze après friction à l'eau (+/- %)** | **18 (2)** | **7 (1)** |

Les résultats montrent une meilleure tenue à l'eau pour la composition J selon l'invention, par rapport à la composition comparative K. L'utilisation d'acide spiculisporique dans une composition comprenant une charge apporte ainsi de la résistance à l'eau.

## Revendications

**1.** Composition cosmétique, notamment de maquillage et/ou de soin de la peau et/ou des lèvres comprenant, dans un milieu aqueux physiologiquement acceptable :

a) au moins 0,01% en poids par rapport au poids total de composition de chitosan natif ayant un poids moléculaire strictement supérieur à 3000 Daltons, cette quantité étant strictement inférieure à 15% en poids, et
b) de l'acide spiculisporique et/ou ses sels.

**2.** Composition selon la revendication 1, dans laquelle le chitosan natif a un poids moléculaire supérieur ou égal à 10 kDa, de préférence supérieur ou égal à 15 kDa, de préférence supérieur ou égal à 20 kDa, de préférence le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

**3.** Composition selon la revendication 1 ou 2, dans laquelle le chitosan natif a un degré d'acétylation du chitosan inférieur ou égal à 80%, de préférence inférieur ou égal à 70%, de préférence inférieur ou égal à 60%, de préférence, inférieur ou égal à 50%, de préférence inférieur ou égal à 35%, de préférence inférieur ou égal à 25%, de préférence inférieur ou égal à 15%.

**4.** Composition selon l'une des revendications précédentes, dans laquelle le chitosan natif est un polysaccharide préparé à partir d'une origine fongique, de préférence issu du mycélium d'un champignon du type Ascomycète, et en particulier d'*Aspergillus niger* et/ou d'un champignon Basidiomycète, et en particulier *Lentinula edodes* et/ou *Agaricus bisporus,* de préférence le champignon est *Aspergillus niger.*

**5.** Composition selon l'une des revendications précédentes, dans laquelle le chitosan natif est présent en une quantité allant de 0,01% à 14% en poids, de préférence de 0,1% à 14% en poids, de préférence de 0,1% à 12% en poids,

de préférence de 0,2% à 7% en poids, de préférence de 0,25% à 5% en poids par rapport au poids total de la composition.

6. Composition selon l'une des revendications précédentes, comprenant en outre au moins un composé choisi parmi les matières colorantes pigmentaires et les charges.

7. Composition selon la revendication 6, dans laquelle la matière colorante pigmentaire est un pigment minéral choisi parmi le dioxyde de titane, les oxydes de fer, les oxydes de zirconium ou de cérium, les oxydes de zinc ou de chrome, le violet de manganèse, le bleu outremer, l'hydrate de chrome et le bleu ferrique, les poudres métalliques comme la poudre d'aluminium et la poudre de cuivre, les nacres, les pigments monochromatiques ; et/ou la charge est choisie parmi les particules de cellulose, les silices, l'amidon, le kaolin, les argiles, les particules de nylon ou de polyméthacrylate de méthyle (PMMA) et leurs mélanges.

8. Composition selon la revendication 6, dans laquelle la matière colorante pigmentaire est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

9. Composition selon la revendication 6 ou 7, dans laquelle la matière colorante pigmentaire est choisie parmi les pigments minéraux, de préférence parmi le dioxyde de titane, les oxydes de fer, les oxydes de zirconium ou de cérium, les oxydes de zinc ou de chrome et leurs mélanges, de préférence le pigment est choisi parmi le dioxyde de titane, les oxydes de fer et leurs mélanges.

10. Composition selon l'une des revendications 6 à 9, dans laquelle la matière colorante pigmentaire est présente en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids.

11. Composition selon l'une des revendications précédentes, dans laquelle l'acide spiculisporique et/ou ses sels est présent dans la composition dans une teneur allant de 0,1% à 25%, de préférence de 0,1 à 15%, de préférence de 0,1% à 10%, de préférence 0,1% à 5%, de préférence 0,1% à 3%, de préférence 0,1% à 2%, préférentiellement de 0,25% à 1,5% et encore plus préférentiellement de 0,5% à 1,5% en poids par rapport au poids total de la composition.

12. Composition selon l'une des revendications précédentes, dans laquelle le milieu aqueux physiologiquement acceptable comprend au moins 5% en poids d'eau par rapport au poids total de la composition, de préférence au moins 10% en poids, de préférence au moins 20% en poids, de préférence au moins 30% en poids, de préférence au moins 40% en poids ; de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50% ; et éventuellement au moins un solvant organique miscible dans l'eau à 25°C choisi parmi les alcools, les polyols et leurs mélanges.

13. Composition selon l'une des revendications précédentes, qui comprend au moins un alpha hydroxy acide, de préférence choisi parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

14. Composition selon l'une des revendications précédentes, qui présente un pH inférieur ou égal à 7, de préférence inférieur ou égal à 6,5, de préférence inférieur ou égal à 6,3, de préférence compris entre 3 et 6,3, de préférence compris entre 4 et 6,3.

15. Procédé de maquillage et/ou de soin de la peau et/ou des phanères, dans lequel on applique la composition selon

l'une des revendications précédentes sur la peau et/ou les phanères.

Europäisches Patentamt
European Patent Office
Office européen des brevets

# RAPPORT DE RECHERCHE EUROPEENNE

Numéro de la demande
EP 23 30 5595

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 03/068824 A1 (KITOZYME S A [BE]; VERSALI MARIE-FRANCE [BE] ET AL.) 21 août 2003 (2003-08-21)<br>* revendications 1-30; exemples 1-10 *<br>----- | 1-15 | INV.<br>A61K8/365<br>A61K8/49<br>A61K8/73<br>A61K8/9728<br>A61Q1/02<br>A61Q19/00 |
| A | US 2021/007959 A1 (LESCH SANDIE [FR] ET AL) 14 janvier 2021 (2021-01-14)<br>* alinéa [0078]; revendications 1-20 *<br>----- | 1-15 | |
| A | WO 03/072610 A1 (COGNIS DEUTSCHLAND GMBH [DE]; SUMSER MARKUS [DE] ET AL.) 4 septembre 2003 (2003-09-04)<br>* page 1, alinéa 3 *<br>* page 13; revendications 1-11; exemples 1-5 *<br>----- | 1-15 | |
| | | | **DOMAINES TECHNIQUES RECHERCHES (IPC)** |
| | | | A61K<br>A61Q |

Le présent rapport a été établi pour toutes les revendications

2

| Lieu de la recherche | Date d'achèvement de la recherche | Examinateur |
|---|---|---|
| Munich | 5 octobre 2023 | Nopper, Agathe |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 23 30 5595

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

05-10-2023

| Document brevet cité au rapport de recherche | Date de publication | Membre(s) de la famille de brevet(s) | Date de publication |
|---|---|---|---|
| WO 03068824 A1 | 21-08-2003 | AT E336516 T1 | 15-09-2006 |
| | | AU 2003215555 A1 | 04-09-2003 |
| | | BE 1014638 A6 | 03-02-2004 |
| | | CA 2475258 A1 | 21-08-2003 |
| | | CN 1642986 A | 20-07-2005 |
| | | DE 60307603 T2 | 13-09-2007 |
| | | DK 1483299 T3 | 27-12-2006 |
| | | EP 1483299 A1 | 08-12-2004 |
| | | ES 2271605 T3 | 16-04-2007 |
| | | JP 2005529191 A | 29-09-2005 |
| | | US 2005130273 A1 | 16-06-2005 |
| | | WO 03068824 A1 | 21-08-2003 |
| US 2021007959 A1 | 14-01-2021 | BR 112020012228 A2 | 24-11-2020 |
| | | CN 111491610 A | 04-08-2020 |
| | | EP 3727310 A1 | 28-10-2020 |
| | | ES 2909734 T3 | 10-05-2022 |
| | | FR 3075049 A1 | 21-06-2019 |
| | | US 2021007959 A1 | 14-01-2021 |
| | | WO 2019121706 A1 | 27-06-2019 |
| WO 03072610 A1 | 04-09-2003 | DE 10208550 A1 | 04-09-2003 |
| | | WO 03072610 A1 | 04-09-2003 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03068824 A
- FR 2679771
- EP 1184426 A


### Littérature non-brevet citée dans la description


- **VAN DE HULST, H.C.** Light Scattering by Small Particles. Wiley, 1957